# EUROPEAN PATENT SPECIFICATION

(11) **EP 2 210 582 B1**
(45) Date of publication and mention of the grant of the patent: **18.09.2019**
(21) Application number: 09151157.6
(22) Date of filing: 22.01.2009
(51) Int. Cl.: A61K 8/64, A61K 8/9706, A61Q 11/00, A61K 36/05

(54) **Dental composition comprising algae and method for producing thereof**
Dental Zusammensetzung mit Algen und Verfahren zur Herstellung davon
Composition dentaire comprenant des algues et procédé pour sa préparation

(43) Date of publication of application: 28.07.2010
(73) Proprietor: Solarvest Bioenergy Inc., Montague PE C0A 1R0 (CA)
(72) Inventor: Wagner, Richard, Bloomington, IN 47401 (US)
(74) Representative: Elsy, David

(56) References cited:
- EP-A- 1 437 124
- EP-B- 1 328 285
- WO-A-99/44573
- US-A1- 2003 022 359
- DATABASE WPI Week 200234 Thomson Scientific, London, GB; AN 2002-295746 XP002540373 & JP 2001 240604 A (LION CORP) 4 September 2001 (2001-09-04)

## Description

### FIELD OF THE INVENTION

The invention relates to a dental composition. More particularly, the invention relates to a dental composition for an animal wherein the composition comprises whole green microalgae.

### BACKGROUND AND SUMMARY OF THE INVENTION

Biofilm, dental plaque, and calculus, etc. are associated with bad breath and with major dental diseases, such as dental caries, gingivitis, periodontitis, and other periodontal diseases, that affect a large percentage of animals each year. Microorganisms, such as bacteria, can contribute to formation of biofilm, plaque, and calculus. Some of the types of microorganisms that are primary, secondary, or tertiary colonizers of biofilm (e.g. dental plaque, calculus, etc.) are *Streptococcus mutans, Streptococcus sanguis, Actinomyces viscosus, Lactobacillus acidophilus, Actinobacillus actinomycetemcomitans, Eikenella corrodens, Porphyromonas gingivalis, Prevotella intermedia, Bacteroides forsythus, Campylobacter rectus, Treponema denticola, Fusobacterium nucleatum, .Capnocytophypa* species, *Tannerella* species, *Peptostreptococcus* species, *Endodontalis* species, and *Escherichia* species.

The formation of biofilm, plaque, and calculus is not only associated with a variety of dental diseases, including dental caries, tooth loss, and periodontitis, but can lead to poor health generally in animals by contributing to the development of systemic diseases of major organ systems such as kidney disease, vascular diseases, and heart disease. Accordingly, there is a need for effective methods of reduction of biofilm, plaque, and calculus on the teeth of animals to improve animal dental health, and to improve animal health generally.

The present invention is directed to the surprising discovery that compositions comprising algae (e.g., green algae) reduce plaque on the teeth of animals. In fact, plaque reduction can occur, for example, by early times (e.g., about 24 hours) after administration of the algae composition. Algae compositions are desirable for use in such dental compositions because algae are natural, non-toxic, non-pathogenic, free of animal pathogens, easy to grow, and resistant to degradation by acids present in the mouth.

In one embodiment of the invention, a composition comprising whole green microalgae is provided, wherein the green microalgae composition is for reducing plaque on at least one tooth of the animal. In this composition embodiment, the algae can be in the form of a paste, a gel, a liquid (e.g., for addition to the drinking water of the animal), a powder, a
freeze-dried composition, a component of an animal treat, a nugget, a dressing for an animal food, an additive for water, a component of a chew product, or a pellet. In another illustrative embodiment, the algae can be freeze-dried and can be capable of being reconstituted.

In another illustrative aspect of the above-described algae composition embodiment, the algae can be genetically modified (e.g., to express an antimicrobial peptide). In yet another embodiment, the algae composition can further comprise one or more of a preservative, an inert carrier (e.g., selected from the group consisting of a paste and a gel), a flavoring, a nutritional carrier, a nutraceutical, a material to enhance adhesion of the algae to at least one tooth of an animal, an antimicrobial (e.g., an antimicrobial peptide or an antibiotic), a plaque-reducing agent, and a calculus-reducing agent.

In still other illustrative embodiments, the algae can be selected from the group consisting of live algae, dead algae, and combinations thereof, or the algae can be of the species *Chlamydomonas reinhardtii.*

In any of the above described embodiments, plaque reduction can occur by about 24 hours after treatment with the algae composition.

### BRIEF DESCRIPTION OF THE DRAWINGS

Figure 1 shows cells of CC-1284 remaining on a molar following coating the molar with an algal paste and successive rinses with deionized water. Panels A-F represent the first through sixth rinses, respectively.
Figure 2 shows cells of CC-1952 remaining on a molar following coating the molar with an algal paste and successive rinses with deionized water. Panels A-F represent the first through sixth rinses, respectively.
Figure 3 shows the quantitative light-induced fluorescence imaging of molars coated with an algal paste from CC-1284 (Panels A and B) and CC-1952 (Panels C and D) before (Panels A and C) and after (Panels B and D) rinsing six times with deionized water.

### DETAILED DESCRIPTION OF THE INVENTION

While the invention is susceptible to various modifications and alternative forms, illustrative embodiments are described herein. It should be understood, however, that there is no intent to limit the invention to the particular forms described, but on the contrary, all modifications, equivalents, and alternatives falling within the spirit and scope of the invention are covered.

As used herein, "biofilm" means a dental biofilm.

As used herein, "calculus" means plaque that has partially or completely calcified.

As used herein, "at least one tooth" means one tooth or more than one tooth of an animal.

As used herein, "whole algae" means algae that have not been extracted (e.g., using a standard cellular extraction method known in the art).

As used herein, the terms "reducing," "reduce," "reduces," and "reduction" in reference to biofilm, plaque, or calculus mean that a lower amount of biofilm, plaque, or calculus, or a combination thereof, is found on at least one tooth of an animal after treatment with the compositions described herein, or that biofilm, plaque, or calculus, or a combination thereof, is completely removed from at least one tooth of an animal after treatment with the compositions described herein, or that biofilm, plaque, or calculus, or a combination thereof, is prevented from forming on at least one tooth of an animal after treatment with the compositions described herein.

As used herein, "green algae" means microalgae from the group Chlorophyta.

As used herein, "algae" means microalgae.

In various embodiments of the methods and compositions described herein, the animal treated with the algae compositions according to the methods described herein can be, for example, a domestic animal, such as a dog, a cat, or a ferret, a wild animal in captivity such as a bear, a panda, a lion, a tiger, a leopard, an elephant, a zebra, a giraffe, a monkey, a chimpanzee, or a gorilla, an equine species (e.g., a race horse), or a human. It should be understood, however, that this list of animals that can be treated using the methods and compositions described herein is non-limiting, and any type of animal in need of treatment with the methods and dental compositions described in this application can be treated.

The invention relates to a composition, More specifically, the invention relates to a composition for an animal wherein the dental composition comprises whole algae. The algae for use in these compositions, kits, articles of manufacture, and methods are green microalgae.

In the above-described embodiments the algae are green algae. In accordance with the invention "green algae" means microalgae from the group Chlorophyta.

In all embodiments, whole algae (e.g., whole algae, either ground algae or algae that has not been ground) can be used in the algal paste, gel, liquid, powder, freeze-dried composition, and other applicable forms that are used in accordance with the invention. The algae can be selected from the group consisting of live algae, dead algae, and combinations thereof.

Exemplary green algae that can be used include *Chlamydomonas* species, (e.g., *Chlamydomonas reinhardtii), Chlorella* species, *Volvox* species, *Dunaliella* species, *Nannochloris* species, *Oedogonium* species, and some marine macrophytes.

*Chlamydomonas reinhardtii* is an exemplary green algae that can be used in the methods and compositions described herein. *Chlamydomonas reinhardtii* grows rapidly and is easy and inexpensive to grow in culture. Exemplary *Chlamydomonas* strains include CC-425, CC-741, CC-744, CC-1284, CC-1952, CC-2137, CC-2277, CC-2519 CC-2677. These strains are available from the Chlamydomonas Center, University of Minnesota (Minneapolis, Minnesota). Exemplary website addresses include http://www.chlamy.org/ chlamydb.html or http://www.chlamy.org/strains.html or http://www.chlamy.org/info.html. Chlamydomonas strains, or other green algae strains, are also available from the ATCC (American Type Culture Collection, P.O. Box 1549, Manassas, VA 20108). An exemplary website address is http://www.atcc.org/ATCCAdvancedCatalogSearch/tabid/112/Default.aspx. Any suitable *Chlamydomonas* strain that can be obtained from the Chlamydomonas Center or the ATCC, or that is known to the skilled artisan can be used.

In illustrative embodiments, the algal cells can be cultured using a variety of techniques known in the art. Typically, algal cultures are supplemented with a carbon source (e.g., glucose or acetate), and the algal cells can be cultured to achieve a desired density. Briefly, standard sterile techniques known to those skilled in the art can be used. In one embodiment, algal cells can be inoculated into standard algal cell culture medium using a laminar flow hood to maintain sterile conditions and the algae can then be cultured with aeration at about 23° C using 18 hour light/6 hour dark cycles. In one illustrative embodiment, progression from small cultures (e.g., 30 ml) to larger cultures (e.g., 200 ml) to large flasks (e.g., 2 liters) is used. The algal cultures are typically stirred to maintain the cells
in suspension. In one embodiment, if the pH of the medium increases to above about 9, for example, the culture medium can be bubbled with CO₂ to reduce the pH to about 7, or an alkaline buffering compound, such as NaHCO₃, can be added. When a desired algal cell density is achieved, an algal cell pellet (e.g., a paste) can be collected by centrifugation, and the supernatant discarded.

In illustrative embodiments, whole algae (e.g., either algae that has been disrupted (e.g., by grinding the algae) or algae that is not disrupted) can be used in the algae paste, gel, liquid, powder, freeze-dried composition, and other applicable forms described herein. The algae can, for example, be collected from culture medium by pelleting the algae by centrifugation and the algal pellet can be used in the compositions described herein. In alternate embodiments, an algal component (e.g., a purified component) or algal components (e.g., a partially purified extract) can be used.

For the embodiments described herein where the algae are genetically modified, exogenous nucleotides (e.g., DNA) can be introduced into the nuclear, chloroplast, or mitochondrial genome of the algae. Nuclear transformation of the algae can be performed, for example, by electroporation essentially as described in Shimogawara, et al., Genetics Vol. 148, 1821-1828, incorporated herein by reference. In another illustrative embodiment, nuclear, chloroplast, or mitochondrial transformation can be achieved using a transgene flanked by homologous nuclear, chloroplast, or mitochondrial targeting sequences that facilitate integration of the transgene into the desired site. See, for example, the chloroplast transformation vector described in WO 2008/021223. In other embodiments, other standard transformation methods known to the skilled artisan can be used including calcium phosphate precipitation, DEAE-dextran transformation techniques, and biolistics. In some embodiments, the molecule expressed (e.g., a peptide, a polypeptide, or a protein) can be expressed at high efficiency (e.g., as described in WO 2008/021223).

In various illustrative embodiments, the algae can be genetically modified to express a plaque reducing agent, a calculus reducing agent, an antimicrobial (e.g., an antimicrobial peptide), and the like. Antimicrobial peptides can include, for example, peptides that inhibit interaction or binding of microbes to components of biofilm, plaque, or calculus, peptides that interfere with the metabolism of microbes, peptides that damage the cell walls of microbes, and the like. Methods of identifying antimicrobial peptides are known in the art and are described in U.S. Patent Nos. 7,465,784 and 7,393,999.

In one embodiment, auxotrophic mutants that can be used (i.e., mutants that differ from the wild-type in requiring one or more nutritional supplements for growth) are readily available at the Chlamydomonas Center, University of Minnesota (Minneapolis, Minnesota; e.g., http://www.chlamy.org/info.html). Such mutants can be genetically complemented by transforming the mutant algae with nucleotides (e.g., exogenous DNA), which facilitates selection of transformed algae containing a desired transgene. This selection method avoids the need for the use of an antibiotic-based selection method.

In one embodiment, the genetically modified algae that express a desired protein, polypeptide, peptide, etc. are further genetically modified such that they will not proliferate in the environment in general, but rather will proliferate only in specific controlled environments (i.e., such strains will not grow or transfer their genes in the wild). In accordance with this invention, such algae are said to be "disabled." Use of such disabled algae strains inhibits or limits spread of the genetically modified algae into the environment.

In one embodiment, disabled strains of algae can be constructed by incorporating a genetic mutation that precludes growth and/or mating outside of a specific controlled environment. For example, the genetically modified algae may be engineered to contain mutations that prevent photosynthesis, and such strains are unlikely to survive in the wild because they cannot produce the energy or reduced carbon necessary to sustain life. An exemplary mutation preventing photosynthesis is a mutation in genes comprising the *psbD*/*psaC* operon of *C*. *reinhardtii,* which is part of the chloroplast genome of *C*. *reinhardtii.* In other embodiments, mutations useful in constructing disabled algal strains are mutations in genes that result in strains that cannot grow in the absence of specific metabolites. These mutant strains are said to be "auxotrophic" for the particular metabolite (e.g, amino acids, vitamins, etc.). In various embodiments, useful mutations require cells to be grown in the presence of arginine, thiamine, or nicotinamide. In yet another embodiment, multiple mutations of the type described above, for example, are combined into a single strain of algae. Combinations of these mutations in a single strain (also called "stacking" of mutations) result in disabled strains that are particularly nonfunctional in growth and mating, and in the ability to grow and transfer their genes in the wild.

In another embodiment, a disabled algal strain takes advantage of mating events. For example, in *C*. *reinhardtii,* when haploid (+) and (-) cells mate to form a diploid cell, only the chloroplast genomes from the (+) mating type organism survive. The chloroplast genomes of the (-) mating type cells are degraded during mating. Accordingly, *C*. *reinhardtii* strains in which the transgene encoding the expressed molecule is located in the chloroplast genome of a (-) cell are advantageous when control of proliferation of transgenic algae is desired. In yet another embodiment, the algal strain can be disabled by mutations in the genes encoding flagella. For example, in *C*. *reinhardtii,* flagella are necessary to hold (-) and (+) cells together during mating. Accordingly, transgene-containing cells with mutations in genes encoding flagella will be unable to transmit the transgene through mating.

In various embodiments of the compositions described herein, the algae can be in the form of a paste, a gel, a liquid (e.g., for addition to the drinking water of an animal), a powder, or a freeze-dried composition (e.g., a composition that is capable of being reconstituted). In one embodiment, the algae can be dried for storage and/or preservation.

In various illustrative aspects, algal pastes can be made by harvesting algal cultures by continuous centrifugation in a batch-centrifuge, where the algal pellet is collected and yields an algal paste. In an alternate embodiment, algae can be harvested by gravitational sedimentation (e.g., in a conical tank). The algal sediment can then be collected and centrifuged to yield the algal paste. In yet another embodiment, the algae may be flocculated from the culture medium by the addition of certain salts, such as ferric chloride, aluminum chloride or aluminum sulfate. The cells that settle out after flocculation may be harvested by centrifugation to generate the algal paste. In yet another embodiment, algae can be concentrated by cross-flow filtration, and then centrifuged to form an algal paste. Other methods for forming an algal paste for use in the methods and compositions described herein are known to those skilled in the art.

In another embodiment, an algal powder can be made by, for example, freeze-drying an algal paste, milling the freeze-dried algae, or grinding the algae in a Waring blender, for example, and then sieving the milled or ground algae composition to form a powder. In one embodiment, the powdered algae can be used directly in the algal compositions described herein for use in treating the teeth of animals. In another embodiment, the powdered algae can be freeze-dried for storage, and reconstituted for use in the algae compositions described herein. In another embodiment, various types of algae forms (e.g. a paste, a powder, a freeze-dried (according to protocols known in the art) algae composition, an algae composition dried in a commercial drier, etc.) can be reconstituted in, for example, a buffer solution or in water to form the liquid algae composition which is used, for example, as an additive for the drinking water of an animal. Algal gel compositions can be made according to procedures well-known to those skilled in the art.

In the above described embodiments of the algal form, the animal can be treated with the algae composition where the algae composition is a component of an animal treat, is in the form of a dressing for animal food, is in the form of a nugget, a pellet, is in the form of an additive for water, or is a component of a chew product (e.g., either an oral or digestible chew product). Any suitable regimen for treatment of the animal with the algae compositions described herein can be used. A person skilled in the art would be readily able to determine a dosing regimen depending on the type of formulation used. Exemplary treatment regimens include application of the algae composition to the teeth of the animal one or more times daily (e.g., one, two, or three times) on weekdays, and one or more times daily on weekends (e.g., one, two, or three times). In another embodiment, a daily regimen is used with application of the algae composition to the teeth of the animal once daily. Further, a staggered regimen, for example, one to three days per week can be used as an alternative to daily treatment, and for the purpose of defining this invention such an intermittent or staggered daily regimen is considered to be within the scope of this invention.

In any of the embodiments described in this application the algae composition can comprise about 90% by weight of algae, about 1% to about 20% by weight of algae, about 15% to about 40% by weight of algae, about 30% to about 60% by weight of algae, about 50% to about 80% by weight of algae, or about 60% to about 100% by weight of algae. In these embodiments, the algae can be whole algae or an extract or purified component of the algae.

In various embodiments of the compositions described herein, the algae compositions can further comprise one or more of a preservative, a flavoring, an inert carrier (e.g., which can be in the form of a paste or a gel), an antimicrobial peptide, an antimicrobial (e.g., an antibiotic), a plaque reducing agent, a calculus reducing agent, a nutritional carrier, a nutraceutical, or other suitable additives.

In another embodiment, preservatives can be added to the algae compositions described herein. Exemplary preservatives include, but are not limited to, Thimersol, benzyl alcohol, methylparaben, propylparaben, benzoic acid, sodium benzoate, and potassium sorbate.

In an additional embodiment a flavoring may be added to the algae composition described herein. Exemplary flavorings include garlic, wood smoke, meat extracts and other meat flavorings, fish extracts and other fish flavorings (e.g., Tuna fish flavoring), fermentation residues, and any combination thereof. In embodiments where the algae composition is for treatment of the teeth of humans, flavorings can include apple, orange, cherry, vanilla, watermelon, bubble gum, apricot, grape, currant, and lemon flavorings, and any combination thereof. In still another embodiment the flavoring may be a sweetening agent, such as saccharin, dextrose, levulose, aspartame, D-tryptophan, dihydrochalcones, sodium cyclamate, sucrose, fructose, glucose, and any combination thereof.

In another illustrative aspect, an oral pharmaceutically acceptable inert carrier can be added to the algae compositions described herein. The inert carrier can be, for example, in the form of a gel, a paste, a powder, or a liquid. Exemplary inert carriers include, but are not limited to, lactose, starch (pharmaceutical grade), dextrin, dicalcium phosphate, calcium sulfate, kaolin, and mannitol.

In yet another embodiment, an antimicrobial peptide can be either expressed in the genetically modified algae that are used in the algae compositions described herein, or can be an additive to the algae compositions described herein, or a combination thereof. Suitable antimicrobial peptides include peptides that inhibit interaction or binding of microbes to components of biofilm, plaque, or calculus, peptides that interfere with the metabolism of microbes, and peptides that damage the cell walls of microbes, and the like. Exemplary methods of identifying antimicrobial peptides are known in the art, and are described in U.S. Patent Nos. 7,465,784 and 7,393,999.

In still another embodiment, antimicrobial agents can be included in the algae compositions described herein. Such agents may include, but are not limited to 5-chloro-2-(2,4-dichlorophenoxy)-phenol, 8-hydroxyquinoline, copper II compounds, phthalic acid, chlorhexidine, alexidine, hexetidine, sanguinarine, benzalkonium chloride, salicylanilide, domiphen bromide, cetylpyridinium chloride, tetradecylpyridinium chloride, N-tetradecyl-4-ethylpyridinium chloride, octenidine, iodine, sulfonamides, bisbiguanides, phenolics, delmopinol, octapinol, and other piperidino derivatives, and nicin preparations, any suitable antibiotics such as augmentin, amoxicillin, tetracycline, doxycycline, minocycline, metronidazole, neomycin, kanamycin, and clindamycin, and any salts of any of these compounds where applicable, and any combinations of these compounds. In another embodiment, antiviral compounds can be included, alone or in combination with any of the above-described antimicrobials. Exemplary anti-viral compounds include cytosine derivatives, purine anaglogues, pyrimidine bases, amantadines, ribavirin, zanamivir, acyclovir, and the like. In yet another embodiment, anti-fungal compounds can be included, alone or in combination with any of the above-described antimicrobials. Anti-fungals agents that are suitable for use in the algae compositions described herein include, but are not limited to, nystatin, miconazole, econazole nitrate, clotrimazole, and flucytosine.

In yet another embodiment, a plaque-reducing agent may be added such as zinc citrate, sanguinarine, baking soda, hydrogen peroxide, stannous salts, copper salts, strontium salts, or magnesium salts, and any combination thereof. Additional exemplary plaque-reducing agents include, for example, fluoride anions. In one embodiment a fluoride anion may be added such as sodium fluoride, stannous fluoride, potassium fluoride, ammonium fluoride, zinc fluoride, germanium fluoride, palladium fluoride, titanium fluoride, sodium fluorozirconate, potassium fluorozirconate, stannous fluorozirconate, stannous fluoroborate, stannous fluorosulfate, sodium fluorosulfate, potassium fluorosulfate, calcium fluorosulfate, mono fluoro phosphate, chlorhexidine, and mixtures thereof. Exemplary fluoride ion-yielding materials can be found in U.S. Patent No. 3,535,421 and U.S. Patent No. 3,678,154.

In another illustrative aspect, a calculus reducing agent may be added such as polyphosphates (including pyrophosphates), polyamino propane sulfonic acid, polyolefin sulfonates, polyvinyl phosphates, polyolefin phosphates, diphosphonates, phosphonoalkane carboxylic acid, polyphosphonates, polyvinyl phosphonates, polyolefin phosphonates, carboxylic acids (including malic, citric and fumaric acids), and sodium hexamethaphosphate and salts of any of these compounds, and any combination thereof.

In yet another illustrative embodiment, a nutritional carrier or supplement may be added to the algae compositions described herein. Exemplary nutritional carriers include minerals, vitamins, oral nutritional supplements, enteral nutritional supplements, and any combination thereof. Minerals that can be used include, but are not limited to, calcium, phosphorus, zinc, manganese, potassium, and any combination thereof. Suitable vitamins include, but are not limited to, vitamins C and D, thiamine, riboflavin, calcium pantothenate, niacin, folic acid, nicotinamide, pyridoxine, cyanocobalamin, para-aminobenzoic acid, bioflavonoids, and any combination thereof. Amino acids can also be added and include, but are not limited to 1-tryptophan, 1-lysine, methionine, threonine, levocarnitine, or 1-carnitine, and any combination thereof. Additional exemplary nutritional supplements include fish oils, in particular fish oils containing large amounts of omega-3 polyunsaturated fatty acids, such as eicosapentaenoic acid and docosahexaenoic acid. Enteral nutritional supplements include, but are not limited to, protein products, glucose polymers, corn oil, and safflower oil. In yet another embodiment, any of these nutritional supplements can be added in any combination.

In another illustrative embodiment, a nutraceutical can be added to the algae compositions described herein. A person skilled in the art would readily be able to determine the type(s) of anti-inflammatory agents useful for the particular animal. Suitable nutraceuticals include anti-inflammatory agents. Exemplary anti-inflammatory agents include non-steroidal anti-inflammatory agents (NSAIDs) including salicylates, propoionic acids, and fenamates, including but not limited to ibuprofen, indomethacin, diclofenac, fenoprofen, piroxicam, nabumetone, aspirin, diflunisal, meclofenamate, mefenamic acid, oxyphenbutazone, phenylbutazone, and acetaminophen. Exemplary steroidal anti-inflammatory agents include corticosteroids, such as hydrocortisone.

In another illustrative aspect, the algae compositions described herein can further comprise such additives as pharmaceutically acceptable diluents, solubilizing agents, a breath enhancing agent, abrasives, polishes, thickening and binding agents, and antioxidants. In various embodiments the algae compositions can include pharmaceutically acceptable diluents (e.g., liquid alcohols, glycols (e.g., polyethylene glycols), glucose solutions (*e*.*g*., 5%), esters, amides, sterile water, buffers (including phosphate or acetate buffers, isotonic saline, and Tris buffers)).

In another embodiment, the solubilizing agents can be, for example, propylene glycol, dipropylene glycol, hexylene glycol, methyl cellosolve, ethyl cellosolve, amyl acetate, ethyl acetate, benzyl benzoate, Tween 80, or Polysorbate 80.

In another embodiment a breath enhancing agent may be added to the algae compositions described herein such as oil of peppermint, oil of wintergreen, oil of spearmint, oil of clove, oil of sassafras, and mixtures thereof.

In various aspects, antioxidants can also be added. Exemplary antioxidants include beta-carotene, vitamin E, vitamin C, vitamin A, tocopherol, butylated hydroxytoluene, butylated hydroxyanisole, tertiary-butylhydroquinone, propyl gallate, ascorbic acid, sodium metabisulfite, uric acid, carotenoids, flavonoids, melatonin, and ethoxyquin.

In any of the above-described embodiments, the algae composition can further comprise a material to enhance adhesion of the algae to at least one tooth of an animal, and the algae can adhere to at least one tooth of an animal for a prolonged period of time, either due to the presence of an agent that enhances adhesion of the algae to the teeth, or due to the adherence characteristics of the algae. Exemplary materials that enhance adhesion of algae to at least one tooth of the animal include carbohydrates and/or carbohydrate-containing materials.

The following examples illustrate specific embodiments in further detail. These examples are provided for illustrative purposes only and should not be construed as limiting the invention or the inventive concept in any way.

### EXAMPLES

### EXAMPLE 1

### CULTURE AND PREPARATION OF ALGAE COMPOSITION

The algal cells can be cultured using a variety of techniques known in the art. Typically, algal cultures are supplemented with a carbon source (e.g., glucose or acetate), and the algal cells can be cultured to achieve a desired density. Briefly, standard sterile techniques known to those skilled in the art can be used. The algal cells can be inoculated into standard algal cell culture medium using a laminar flow hood to maintain sterile conditions and the algae can then be cultured with aeration at about 23° C using 18 hour light/6 hour dark cycles. Progression from small cultures (e.g., 30 ml) to larger cultures (e.g., 200 ml) to large flasks (e.g., 2 liters) can be used. The algal cultures are typically stirred to maintain the cells in suspension. If the pH of the medium increases to above about 9, the culture medium can be bubbled with CO₂ to reduce the pH to about 7, or an alkaline buffering compound, such as NaHCO₃, can be added. When a desired algal cell density is achieved, an algal cell pellet can be collected by centrifugation, and the supernatant discarded.

### EXAMPLE 2

### PLAQUE REDUCTION IN CATS

Experiments were conducted with *C*. *reinhardtii* to determine if the oral delivery of the algae as a paste to the surface of the teeth of cats is an effective means to reduce plaque accumulation. Algal strains CC-741 and CC-2677 were grown for 1 week in modified Tapp media (Harris, 1989) in 20 liter carboys and harvested using a TZ28 Sorval rotor at 16,000 RPM at 4° C. Approximately 200 liters (10 carboys) were harvested for each strain. The algal paste was collected from the rotor and stored at 4° C for approximately 1 week. The algal paste was packed into a 10 ml syringe and 1 cc of the paste was delivered to the teeth of 5 cats. Following delivery of the paste, the paste was dispersed on the surface of the teeth by manually massaging the animals' cheeks.

The results from the experiments show that plaque accumulation on the teeth of animals treated with the algal paste of strain CC-741 was less than plaque accumulation on the teeth of the control group. Four of the five animals treated with the algal paste show a reduction in the amount of plaque by 24 hours after treatment. In contrast, 3 of the 5 control animals show an increase in plaque accumulation during the same time period. One animal (Laurel) in the control group appears to deviate substantially from the response of the animals, showing a precipitous decrease in plaque accumulation. The data from the trial is provided in Tables 1, 2, 3, and 4. The results show a 24% reduction in plaque for animals treated with *C*. *reinhardtii* strain CC-741 (Table 1; Group A) versus controls (Table 2; Group B). The results for *C*. *reinhardtii* strain CC-2677 are shown in Tables 3 and 4. *C. reinhardtii* strain CC-741 is a walled algal strain and *C*. *reinhardtii* strain CC-2677 lacks cell walls.

**Table 1. Plaque evaluation following treatment of feline teeth with C. reinhardtii strain CC-741 (Group A)**

| **Cat** | **Baseline** | **24 hour** |
|---|---|---|
| Grace | 6.33 | 5.17 |
| Magpie | 7.17 | 5.67 |
| Aruba | 7.00 | 5.00 |
| Lilac | 3.50 | 3.50 |
| Shirley | 5.83 | 3.33 |
| **Mean** | 5.97 | 4.53 |
| **S.D.** | 1.48 | 1.05 |
| **% Change** | | 24% |

**Table 2. Plaque evaluation following treatment of feline teeth with deionized water (Group B)**

| **Cat** | **Baseline** | **24 hour** |
|---|---|---|
| Lily | 10.00 | 10.33 |
| Laurel | 8.67 | 4.5 |
| Kitt | 3.33 | 4.16 |
| Bora Bora | 8.33 | 8.67 |
| Audrey | 4.50 | 4.33 |
| **Mean** | 6.97 | 6.40 |
| **S.D.** | 2.88 | 2.89 |
| **% Change** | | 8% |

**Table 3. Plaque evaluation following treatment of feline teeth with C. reinhardtii strain CC-2677 (Group A)**

| **Cat** | **Baseline** | **24 hour** |
|---|---|---|
| Sage | 11.8 | 8.67 |
| Robin | 7 | 7.5 |
| Maui | 6.33 | 8.67 |
| Bora Bora | 6.17 | 9.5 |
| **Mean** | 7.83 | 8.59 |
| **S.D.** | 2.67 | 0.82 |
| **% Change** | | 10% |

**Table 4. Plaque evaluation following treatment of feline teeth with deionized water (Group B)**

| **Cat** | **Baseline** | **24 hour** |
|---|---|---|
| Figi | 2.5 | 2.67 |
| Judy | 8.5 | 9 |
| Chickadee | 7.5 | 4.8 |
| Finch | 7 | 5.67 |
| **Mean** | 6.38 | 5.54 |
| **S.D.** | 2.66 | 2.63 |
| **% Change** | | 13% |

### EXAMPLE 3

### PLAQUE REDUCTION IN DOGS

Thirty healthy, purpose-bred beagle dogs 1 year of age or older, with normal dental occlusion, received a full dental scaling and polishing of their teeth under general anesthesia (Day -7) (7 days before the start of the testing). On Day 0 and Day 14 each animal was placed under short acting anesthesia to evaluate the plaque indices using a modified Quigley and Hein (1962) (Turesky, 1970) plaque index after the application of a disclosing agent to the teeth followed by rinsing to remove residual excess. The teeth were visually halved horizontally into gingival and occlusive halves that were each given a score for the percentage of coronal surface covered with plaque and the thickness of plaque. The score for the tooth was obtained by adding the gingival and occlusive scores for each half (thickness multiplied by coverage). The sum of the tooth scores on one side of the jaw was termed "the total tooth score" and the whole mouth mean score is the average of the total tooth scores for each animal. On Day 0 each beagle was stratified according to high, medium and low plaque accumulation and then randomly assigned equally to either the treatment and control group. An average volume of 7 ml of *Chlamydomonas reinhardtii* concentrated algal paste, harvested live, approximately 10⁷/ml, strain CC-741 wild-type was applied topically with a sterile 12 cc syringe daily, 2 hours after feeding, to the teeth of each beagle in the treatment group. The control beagles received no treatment. All dogs in the study were fed a standard dry diet. Daily application of algae paste resulted in a 6.8% (SD 2.1) reduction in mean plaque scores compared with control beagles that received no treatment. Table 5 demonstrates that when the plaque scores are rank ordered, the paired comparisons showed the same plaque scores for the highest 6 beagles in the treatment and control groups, while the remaining 9 beagles in the treatment group all had lower scores than their comparative control group ranked pair. Further, the treatment group had the lowest overall plaque scores of 5.5 (2 beagles) compared to the lowest score of 6.6 in the control group. These data indicate that *Chlamydomonas* algal paste contacting the teeth of beagles reduces plaque formation.

**Table 5. Plaque scores in Beagles (control and treatment groups)**

| **Dog** | **Control** | **Treatment** |
|---|---|---|
| 1 | 12.1 | 12.1 |
| 2 | 10.9 | 10.9 |
| 3 | 10.8 | 10.8 |
| 4 | 9.5 | 9.5 |
| 5 | 9.2 | 9.2 |
| 6 | 9.0 | 9.1 |
| 7 | 8.9 | 8.4 |
| 8 | 8.8 | 8.3 |
| 9 | 8.7 | 7.1 |
| 10 | 8.6 | 6.9 |
| 11 | 7.4 | 6.8 |
| 12 | 7.3 | 6.5 |
| 13 | 7.2 | 6.2 |
| 14 | 6.7 | 5.5 |
| 15 | 6.6 | 5.5 |
| **Average** | 8.8 | 8.2 |
| **Stand. Dev.** | 1.6 | 2.06 |

### EXAMPLE 4

### PLAQUE REDUCTION ASSAY

a. General Test Design. To evaluate the various algae preparations a series of at least 3 screening tests will be performed to identify preparations for the prevention of dental plaque formation in animals. The screening tests will be designed as 7-day longitudinal studies with parallel groups of 5-10 animals. This will be followed by a complete crossover test to demonstrate the anti-plaque potential of the preparations selected from the screening tests.
b. Test Procedures. Each screening test will be designed as a one-week (7-day) clean tooth longitudinal test design. The test agents will be applied to the animals' teeth 3 times daily M-F and 2 X daily on the weekends with applications separated by at least 2 hours. Experimental procedures will be conducted using GLP guidelines. The animals will be fed a nutritionally complete commercially-available dry dog food (Purina Dog Chow) at approximately 10:00 a.m. daily.

Prior to the initiation of each study the animals will be given a complete dental prophylaxis to remove all exogenous deposits (plaque, calculus, stain, debris) from the surfaces of their teeth. On the day each screening study will begin, the animals will be given Atropine (0.04 mg/Kg) by s.c. injection 20 minutes prior to the anesthesia. A certified laboratory animal technician will anesthetize the dogs with Butorphanol (0.15 mg/Kg) and Medetomidine (0.025 mg/Kg) I.M. followed by Ketamine HCL (5.0 mg/Kg) I.M. The animals will then be given a thorough prophylaxis. Following the dental prophylaxis, the animalss' teeth will be imaged using the QLF System. The animals will then be given Atipamezole (0.05 mg/Kg) I.M. as an anesthesia reversal. The animals will be evenly stratified by block design into the desired number of groups of at least 5 animals per group. The animals will be balanced on the basis of plaque data obtained from the most current plaque-forming rate study. Considering the number of preparations to be evaluated, it is expected that each screening test will involve 5 groups with 5 or 6 animals per group. The test materials will be topically applied individually to the animals' teeth as an aqueous slurry using a syringe. Three (3) treatments will be given on week-days (Monday - Friday) and two (2) treatments will be administered on Saturday and Sunday; only one treatment will be given on the day the animals are prophied. The treatments will be performed at approximately the same time every day with 2-hour intervals between treatment applications. Each treatment group will have a coded beaker, which is designated for that treatment only. Each test group will have a color-coded tag attached to the animal's cage to correspond with the coded test group. The test solutions will be applied to all of the maxillary and mandibular teeth in their assigned treatment group. A 5cc syringe will be used to apply the solution. Specifically, 2.5cc of each test solution (within the appropriate group) will be evenly dispersed to each hemi jaw over the teeth to be evaluated and allowed to pool in the mandibular region. No attempt will be made to prevent the animals from swallowing the solutions. Care will be taken to prevent the disturbance of naturally occurring plaque. On the designated examination day, 7 days after initiation of the treatment regimen, the animals will be examined by block in a random sequence to avoid systematic bias. The animals will be anesthetized as previously stated, taken to the examination area by a certified laboratory animal technician.

The animals will then have their teeth imaged using Quantitative Laser/Light Fluorescence (QLF). The animals will then be examined for oral malodor, and disclosed plaque. Examiner observations will be recorded on prepared exam forms by the recorder who is not directly involved in the examinations. Baseline Quantitative Laser Florescence exams will be done immediately following the dental prophylaxis and after 7-days while the animals are still under anesthesia. The animals will be examined in a random sequence to avoid systematic bias. The examinations will be performed independently by an experienced QLF examiner. Specifically a hand piece utilizing a high intensity halogen light source, a video camera and a mirror for focusing, will be placed on the buccal side of the each of the following teeth:

| | |
|---|---|
| Maxilla: | I₃, C, P₂, P₃, P₄, M₁ |
| Mandible: | C, P₂, P₃, P₄, M₁ |

The QLF hand piece is coupled to a computer to permit visual viewing and recording of the images. The distance and angle of the QLF hand piece will remain essentially constant for each tooth imaged using the video repositioning (VidRep) program at 90-95%. A Halimeter will be used to measure Volatile Sulfur Compounds (VSC). The sampling tube will be placed parallel to the buccal Maxillary P₄. Cheek mucosa will be kept away from the end of the sampling tube and the animal's mouth closed. The highest reading after a 10-second period will be recorded. Both right and left sides will be sampled. The score for the animal is the mean of these two readings.

For the visual clinical dental plaque examinations the plaque will be disclosed by applying the undiluted disclosing solution (Red Cote; John O. Butler Company; 1.5% D&C Red No. 28) to the buccal surface of each tooth with a syringe and immediately rinsing with water. The gingival and occlusal half for each tooth will be scored. The teeth that will be examined will be:

| | |
|---|---|
| Maxilla: | I₃, C, P₂, P₃, P₄, M₁; |
| Mandible: | C, P₂, P₃, P₄, M₁ |

The amount of the buccal surface covered with dental plaque will be visually graded using the following scale: 0 = no observable plaque; 1 = scattered plaque covering less than 24% of the tooth surface; 2 = plaque covering between 25 and 49% of the tooth surface ; 3 = plaque covering between 50 and 74% of the tooth surface; 4 = plaque covering more than 75% of the tooth surface. The thickness of the plaque will be estimated using the following scale: 1 = Light; 2 = Moderate; and 3 = Heavy. The clinical plaque score will be a function of both the coverage and the thickness. The coverage score is multiplied by the intensity factor to give a gingival and occlusal score for each tooth. The gingival and occlusal values for each tooth are added together to obtain a tooth sum. The score for each animal is the mean sum for all teeth scored. These same procedures will be used for the complete crossover study. For the first leg of the study the animals will be randomized to either the test or placebo treatment and examined after the 7-day test period. The animals will then have a one-week "washout" period and then provided another dental prophylaxis followed by the initiation of the alternative treatment regimen. In this manner all animals will receive both the test and placebo treatments with the test concluded after the second leg or test period.

### EXAMPLE 5

### PLAQUE REDUCTION ASSAY

*Chlamydomonas reinhardtii* concentrate live or freeze dried, or in combination with synergistic agents and various application methodologies will be used. This phase will run for a total of 7 days. Dogs chosen for use on the study will have their teeth scaled and polished upon initiation of the test (Day -7). Each animal will have plaque indices scored on Day 0 before dental cleaning. The scores will indicate the rate of plaque buildup for each animal when being fed only a standard diet. This Pre-Test information will be used to designate the animals in one of three groups so as to stratify the group as a whole to reduce variability for the Testing Period.

Day 0 through Day 14 will be the testing period in which the test article, along with a standard diet, will be offered on a daily basis to the dogs within the test group for evaluation of plaque buildup as compared to evaluation of the control group which will be offered only a standard diet.

A minimum of 20 animals (e.g., Purpose-bred Beagle dogs) 1 year of age and older will be used. Groupings will be determined by evaluating the animals' individual scores obtained from the pre-testing period. Based on individual scores, 10 dogs will be in the test group and 10 dogs will be in the control group. Each dog will have a unique ear tattoo and cage card for identification.

Healthy adult dogs meeting the above description will be eligible for the study. Before the start of the study, dogs will undergo an oral cavity examination for normal occlusion and presence of all test teeth. Dogs will be pair-housed as intended for this study prior to study initiation. Individual body weights will be measured and recorded upon initiation of the study, weekly throughout the study, and upon completion of the study. Standard colony diet will be meal-fed, checked daily, and supplied in appropriate amounts according to body weight prior to study initiation.

Dogs in the test group will receive the standard diet for one hour and the test article will be applied two hours after the standard diet is removed from the dog's enclosure. The volume of test article required is 10 milliliters per treatment dog per day to be topically applied with a sterile 12 cc syringe. The control group will not receive anything in addition to the standard diet. Food consumption will be recorded daily throughout the study.

A veterinarian will give a complete physical examination to all dogs prior to Day -7. Each dog will be evaluated as to general health, body and hair coat condition, and comments will be recorded. Two milliliters of blood will be taken from each dog to test for BUN, PCV, and TP at the physical examination if this data has not been updated within the last 6 months.

Each animal will have its teeth scaled and polished under general anesthesia on Day -7. Each animal will have a plaque index scored at Day 0 using sedation or short-acting anesthesia. The extent of plaque is to be recorded after staining with a disclosing agent and rinsing the tooth surface. The dogs will be offered the standard diet only during this time period.

Each animal will have its teeth scaled and polished under general anesthesia on Day 0. On Day 14, each animal will have plaque evaluated using sedation or short-acting anesthesia if needed. The examination of teeth will include the buccal surfaces and both sides of the mouth. Review will be limited to these nine teeth: upper jaw - incisor 3 (13), canine (C), premolar 3 (P3), premolar 4 (P4), molar 1 (M1); lower jaw - canine (C), premolar 3 (P3), premolar 4 (P4), molar 1 (M1).

Oral malodor will be evaluated on Day 0 and Day 14 by using a Halimeter. Readings will be obtained by putting the straw end of the Halimeter into the dog's mouth, between the cheek and jaw. A pocket will be made and the lips will be closed around the straw to assure accurate readings. One reading will be taken on each side of the mouth, and will be recorded in ppb.

Plaque will be evaluated using a modification of the Quigley and Hein (1962) (Turesky, 1970) plaque index. The extent of plaque and plaque thickness will be determined by placing a disclosing agent on the teeth and rinsing the excess off with water. The teeth are visually halved horizontally into gingival and occlusal halves. Each half is given a score for the percentage of coronal surface covered with plaque and thickness of plaque. The score for each half is calculated by multiplying the coverage and thickness scores. Gingival and occlusal scores are then added together for a tooth score. The sum of the tooth scores on one side of the jaw is termed the total tooth score, and the whole mouth mean is calculated by averaging the total tooth scores for each animal.

**Table 6. Plaque Scoring Method**

| **PLAQUE SCORING METHOD** | | |
|---|---|---|
| **Score** | **% of Plaque Coverage** | |
| 0 | No plaque detected | |
| 1 | 1 - 24 | |
| 2 | 25 - 49 | |
| 3 | 50 - 74 | |
| 4 | 75 - 100 | |

| **Score** | **Plaque Thickness** | **Disclosing Agent Color** |
|---|---|---|
| 1 | Light | Pink to Light Red |
| 2 | Medium | Red |
| 3 | Heavy | Dark Red |

Qualified personnel will perform clinical observations twice daily in accordance with institutions animal care observation standards. All animals will be evaluated twice daily with reference to protocol for (Recognizing Pain, Stress, and/or Distress). Clinical laboratory diagnostic procedures will be performed as needed. Veterinary care will be given as appropriate to each individual animal in accordance with the Program of Veterinary Care.

Test Article may vary by each individual trial. Test article consistency will be sticky paste/gel that will be drawn from a container into sterile syringes. The test article will then be administered onto the teeth via syringe. The test article will be stored in refrigeration for the duration of the study.

With the exception of drugs used to anesthetize the animals for dental scoring and cleaning, vaccinations, drugs, and locally applied or lickable antiseptics should not be used for the length of the study. If an individual animal requires treatment as determined by the veterinarian, the Sponsor will be notified and the necessity of removing the animal from the study will be determined. The study will be stopped if the average weight change of the dogs is greater than -10%. If any dog loses more than 15% of its base (initial) weight, that dog will be evaluated by the veterinarian and removed from the study if the veterinarian deems it necessary to do so. The study may be stopped at any time at the veterinarian's discretion if the animals' health is at risk. A veterinarian will perform a necropsy on any animal found dead or euthanized to determine the cause of death or illness.

Variables to be measured will include the effect of *Chlamydomonas reinhardtii* on the formation of dental plaque, the effect of *Chlamydomonas reinhardtii* on the formation of dental malodor, and the effect of *Chlamydomonas reinhardtii* on the formation of dental calculus. Other variables to be measured include food consumption, body weights, and adverse reactions through daily observations. Statistical analysis will include individual t-tests, mean scores, and standard error functions.

### EXAMPLE 6

### MOLARS TREATED WITH ALGAL PASTE

A total of 6 extracted human molars (a tooth in each group) were used for this study. These specimens were removed of soft tissue. A window was made on the surface of sound enamel specimen by nail varnish. These specimens were immersed into demineralizing solution (White, 1987) at 37 °C for 2 hours to produce the early artificial white-spot lesions. The whole surface of each molar was covered with an algal strain. After 5 minutes, each molar was shaken in a water bottle for 30 seconds and any surplus alga was rinsed off. The images of these specimens were acquired with a standard white-light dissecting microscope. Digital images of the molar prior to treatment and after rinsing were recorded. After the 30 second rinse, strains CC-1284, CC-741, and Phyvac WSSV-VP28 were not evident on the enamel surface lesion, suggesting a low level of enamel adherence. Strains CC-1284 and CC-2677 adhered to parts of the surface whereas CC-1952 covered the whole surface of the molar.

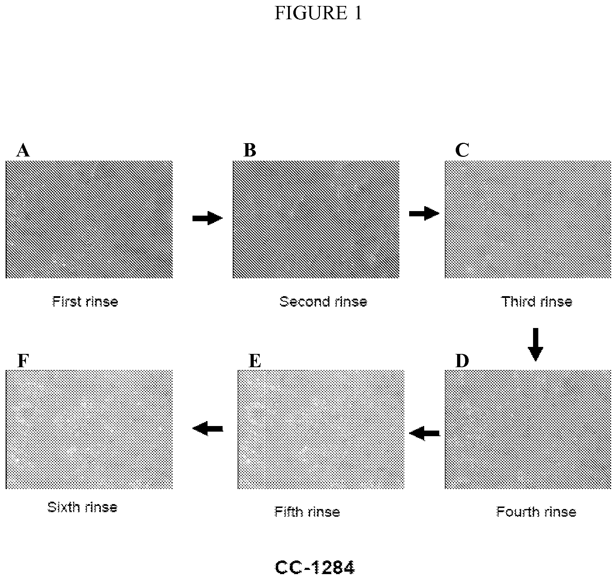

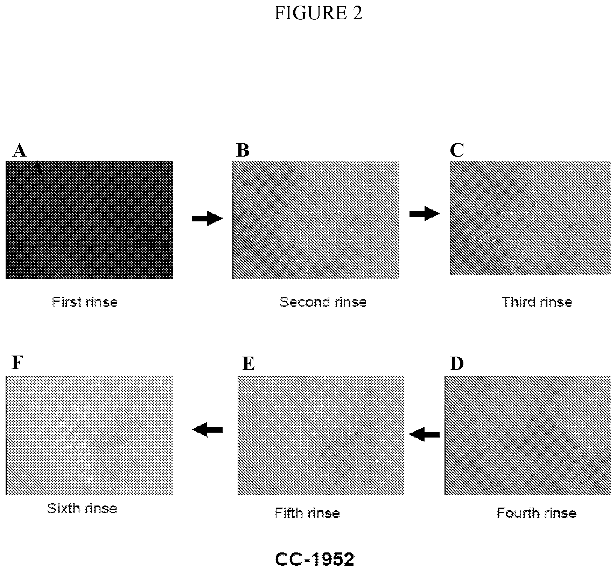

The experiment was repeated again, with the rinsing treatment repeated 6 times. CC-2677 was removed from the surface after the second rinse. CC-1284 and CC-1952 could not be removed after the sixth rinse, although adherence of strain CC-1952 to the tooth surface was greater than for CC-1284 (Figures 1 and 2).

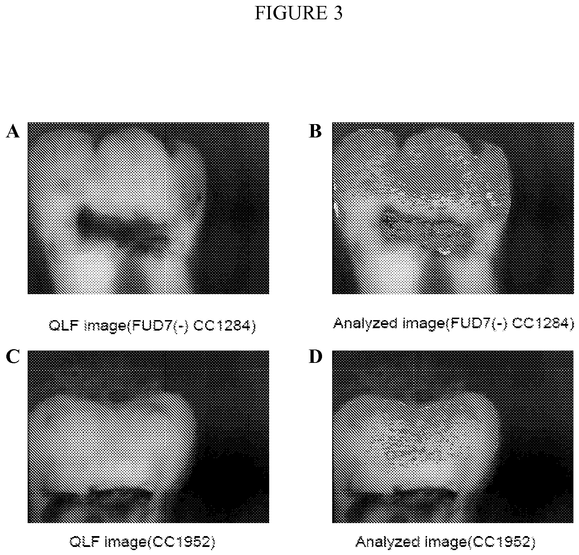

The rinsing experiment was repeated a third time with CC-1284 and CC-1952. After the sixth rinse, digital images of specimens were made using the Quantitative Light-induced Fluorescence system (Inspektor-Pro 2.0.0.32, Inspektor Research System B.V., Netherlands). These images were analyzed with the measurement for red fluorescence using Inspektor-Pro 2.0.0.32. QLF images and its analyzed images are shown in Figure 3. From these images it was calculated that after the sixth rinse, 16.60 mm² and 45.60 mm² of the molar surface was covered with cells of CC-1284 and CC-1952, respectively.

### EXAMPLE 7

### TOOTHPASTE

An exemplary animal toothpaste formulation for use in the compositions and methods described herein is below. Sample ingredients for the toothpaste are as follows (expressed as percent by weight): dental grade silica abrasive at about 10-12% (gives polishing abrasion and thickens toothpaste); dicalcium phosphate dihydrate at about 2-4% (dentifrice grade toothpaste polish and abrasive); pumice (a toothpaste polish and 1-2% abrasive); toothpaste thickening and binding agents, such as sorbitol at about 10-40%, polyethylene glycol at about 4%, carboxymethylcellulose at about 1%, glycerin at about 15%, flavor at about 1-3%, the algae composition at about 5% (or another effective amount depending on the intended application), and water sufficient for processing. Toothpaste formulations for humans may also include surfactants (cleaning and foaming agents). Toothpaste formulations for animals other than humans typically do not include surfactants. The formulation may also include about 2% sodium fluoride in a suitable form.

### EXAMPLE 8

### CHEW TREAT

The algae composition as described herein may be added to raw hide as a chew treat to improve animal dental health. The raw hide can be made from cattle hides, horse hides, sheep skins, goat skins, buffalo hides or pig skins, for example. The raw hide may be cut into sections or strips. Alternatively, the raw hide sections may be ground into smaller pieces of about 1/8 inches to about 1/2 inches in diameter, for example, and dried. The algae composition may be sprayed onto the dried raw hide pieces. In this embodiment, the pieces can be placed in a rotating mixing drum and the algae composition sprayed onto the tumbling pieces or chews. Following the coating (spraying) step, the product will be placed on screens in racks and air dried. The resulting chew treats containing the algae composition may be molded into the shape of a animal chew treat.

Another exemplary approach is to mix the algae composition and dried raw hide with meat meal, bone meal, a binder such as malotdextrin, and/or gelatin. Water will be added in a quantity sufficient for processing. The formula will be heated, mixed, and placed into molds. After molding into suitable shapes, the product will be dried in an oven.

### EXAMPLE 9

### TOOTHPOWDER

An exemplary powder formulation for use in the compositions and methods described herein is provided. According to the following formulation (components expressed as percent by weight), the ingredients are mixed well to make a fine powder, including calcium carbonate at about 75.0%, glycerin at about 10.0%, flavor at about 1.0%, propylparaben at about 0.005%, sodium laurylsulfate at about 1.3% (typically used only for formulations for humans), saccharin sodium at about 0.1 %, the algae composition at about 5% (or another effective amount depending on the intended application), and water q.s. total 100%.

An additional exemplary toothpowder formulation with fluoride will be made by mixing together the following ingredients (components expressed as percent by weight), in the indicated proportions: microcrystalline aluminum hydroxide at about 86%, aluminum hydroxide (325 mesh) at about 5.00%, flavoring matter at about 0.60%, saccharin (soluble) at about 0.25%, sodium fluoride at about 0.10%, the algae composition at about 10%, and water.

## Claims

1. A composition comprising whole green algae for use in reducing plaque on at least one tooth of an animal, wherein the green algae is microalgae, and wherein the green algae is selected from the group consisting of *Chlamydomonas, Chlorella, Volvox, Dunaliella, Nannochloris* and *Oedogonium.*

2. The compositions of claim 1, wherein the algae are in the form of a paste, gel, liquid, nugget, powder, pellet, dressing for animal food or an additive for drinking water or are a component of an oral or digestible treat or chew product.

3. The composition according to any preceding claim, wherein the algae are freeze-dried and are capable of being reconstituted.

4. The composition of any preceding claim, wherein the algae are genetically modified.

5. The composition of claim 4, wherein the algae are genetically modified to express an antimicrobial peptide.

6. The composition according to any preceding claim, wherein the algae composition further comprises a preservative, flavouring, inert carrier to nutritional carrier.

7. The composition of claim 6, wherein the inert carrier is selected from a paste and a gel.

8. The composition according to any preceding claim, wherein the algae composition further comprises a material to provide adhesion of the algae to that at least one tooth.

9. The composition according to any preceding claim, wherein the algae composition further comprises an antimicrobial peptide or other antimicrobial, an antibiotic, an additional plaque-reducing agent or an anti-calculus agent.

10. The composition according to any preceding claim, wherein the algae are selected from live algae, dead algae, and combination thereof.

11. The composition according to any preceding claim, wherein the algae are from the species *Chlamydomonas reinhardtii.*

12. The composition of any preceding claim, wherein the animal is a dog or a cat.

13. The composition according to any preceding claim, wherein the algae adhere to the at least one tooth.

## Patentansprüche

1. Zusammensetzung enthaltend ganze Grünalgen zur Verwendung bei der Reduzierung von Plaque an mindestens einem Zahn eines Tieres, wobei die Grünalge eine Mikroalge ist und wobei die Grünalge ausgewählt ist aus der Gruppe bestehend aus *Chlamydomonas, Chlorella, Volvox, Dunallella, Nannochloris und Oedogonium.*

2. Zusammensetzung nach Anspruch 1, wobei die Algen in Form einer Paste, eines Gels, einer Flüssigkeit, eines Klumpens, eines Pellets, eines Dressings für Tiernahrung oder eines Trinkwasserzusatzes oder als Komponente eines oralen oder verdaulichen Behandlungs- oder Kauprodukts vorliegen.

3. Zusammensetzung nach einem der vorhergehenden Ansprüche, wobei die Algen gefriergetrocknet und rekonstituiert sind.

4. Zusammensetzung nach einem der vorhergehenden Ansprüche, wobei die Algen genetisch verändert sind.

5. Zusammensetzung nach Anspruch 4, wobei die Algen zur Expression eines antimikrobiellen Peptids genetisch verändert sind.

6. Zusammensetzung nach einem der vorhergehenden Ansprüche, wobei die Algenzusammensetzung ferner einen Konservierungsstoff, einen Geschmacksstoff, einen gegenüber einem Nahrungsträger inerten Träger enthält.

7. Zusammensetzung nach Anspruch 6, wobei der inerte Träger ausgewählt ist aus einer Paste und einem Gel.

8. Zusammensetzung nach einem der vorhergehenden Ansprüche, wobei die Algenzusammensetzung ferner ein Material enthält, das eine Haftung der Algen an dem wenigstens einen Zahn ermöglicht.

9. Zusammensetzung nach einem der vorhergehenden Ansprüche, wobei die Algenzusammensetzung ferner ein antimikrobielles Peptid oder ein anderes antimikrobielles Mittel, ein antibiotisches, ein zusätzliches plaquereduzierendes Mittel oder ein Mittel gegen Zahnstein enthält.

10. Zusammensetzung nach einem der vorhergehenden Ansprüche, wobei die Algen ausgewählt sind aus lebenden Algen, toten Algen und einer Kombination derselben.

11. Zusammensetzung nach einem der vorhergehenden Ansprüche, wobei die Algen aus der Gattung *Chlamydomonas reinhardtii* stammen.

12. Zusammensetzung nach einem der vorhergehenden Ansprüche, wobei das Tier ein Hund oder eine Katze ist.

13. Zusammensetzung nach einem der vorhergehenden Ansprüche, wobei die Algen an dem wenigstens einem Zahn haften.

## Revendications

1. Composition renfermant des algues vertes complètes destinée à être utilisée pour réduire la plaque dentaire sur au moins une dent d'un animal, l'algue verte étant une microalgue et étant choisie dans le groupe formé par les algues vertes suivantes :
*Chlamydomonas, Chlorella, Volvox, Dunaliella, Nannochloris et Oedogonium*

2. Composition conforme à la revendication 1,
dans laquelle
les algues sont sous la forme d'une pâte, d'un gel, d'un liquide, de pépites, d'une poudre, de pastilles conditionné(es) pour un aliment animal ou un additif pour de l'eau de boisson ou constitué(es) d'une friandise ou un composé d'un produit de mâchage ou oral ou digestible.

3. Composition conforme à l'une quelconque des revendications précédentes,
dans laquelle
les algues sont séchées par congélation et sont susceptibles d'être reconstituées.

4. Composition conforme à l'une quelconque des revendications précédentes,
dans laquelle
les algues sont génétiquement modifiées.

5. Composition conforme à la revendication 4,
dans laquelle
les algues sont génétiquement modifiées pour exprimer un peptide antimicrobien.

6. Composition conforme à l'une quelconque des revendications précédentes,
dans laquelle
la composition à base d'algues renferme en outre un support inerte de conservation de saveur du support nutritionnel.

7. Composition conforme à la revendication 6,
dans laquelle
le support inerte est choisi parmi une pâte et un gel.

8. Composition conforme à l'une quelconque des revendications précédentes,
dans laquelle
la composition à base d'algues renferme en outre un matériau permettant aux algues d'adhérer à la dent.

9. Composition conforme à l'une quelconque des revendications précédentes,
dans laquelle
la composition à base d'algues renferme en outre un peptide antimicrobien ou un autre agent antimicrobien, un antibiotique, un agent de réduction de la plaque dentaire supplémentaire ou un agent antitartre.

10. Composition conforme à l'une quelconque des revendications précédentes,
dans laquelle
les algues sont choisies parmi des algues vivantes, des algues mortes et leur combinaison.

11. Composition conforme à l'une quelconque des revendications précédentes,
dans laquelle
les algues sont des algues de l'espèce *Chlamydomonas reinhardtii.*

12. Composition conforme à l'une quelconque des revendications précédentes,
dans laquelle
l'animal est un chien ou un chat.

13. Composition conforme à l'une quelconque des revendications précédentes,
dans laquelle
les algues adhèrent à la dent.
